# EUROPEAN PATENT APPLICATION

(11) **EP 4 060 672 A1**
(43) Date of publication of application: **21.09.2022**
(21) Application number: 21163329.2
(22) Date of filing: 18.03.2021
(51) Int. Cl.: G16B 20/50, G16B 40/20

(54) **METHOD FOR PREDICTION OF GENETIC MUTATION AND RECOMBINATION OF VIRUSES WITH DEEP LEARNING**

(71) Applicant: Jacobs, Ivan, 7321 Luxembourg Steinsel (LU)
(72) Inventor: Jacobs, Ivan, 7321 Luxembourg Steinsel (LU)

(57) **Abstract**

There are two major sources of change in nucleotide sequences over time: mutation and recombination. Mutation occurs when a mistake during replication or a physical or chemical assault changes a nucleotide to something different from that in the ancestral genome. Cellular DNA polymerases have editing functions that keep the intrinsic rate of nucleotide substitutions very low, but many viral polymerases have no editing functions. Editing refers to the process of excising a misincorporated nucleotide and replacing it with the correct nucleotide during replication. Because the viral polymerases lack editing functions, viruses that rely on viral polymerases accumulate mutations at a much higher rate per generation than cells do.

Recombination occurs when two viruses co-infect the same cell and an offspring virus contains a combination of genes that originate with both parents instead of just one parent. These are larger-scale genetic changes than those typically classified as mutations. They may enable viruses to jump from species or result in a pandemic because an adaptive immune response against one of the parent viruses is not effective against the recombinant offspring.

Mutations and recombination causing for large genetic variations of viruses result in difficulties to create sustainable treatment. The invention discloses a method to predict possible new genetic variants of viruses.

## Description

### Technical Field

The present disclosure relates, amongst others, to methods and systems for analysis and prediction of the genetic epidemiology of viral infectious diseases such as but not limited to coronavirus and influenza. By using deep learning methods the invention is specifically capable of predicting genetic diversity arisen through mutation and recombination of viruses that cause infectious diseases. The invention allows the design, optimization and assessment of the efficiency of vaccines based upon prediction of virus mutations.

### Background Art

There are two major sources of change in nucleotide sequences over time: mutation and recombination.

Mutation occurs when a mistake during replication or a physical or chemical assault changes a nucleotide to something different from that in the ancestral genome. Cellular DNA polymerases have editing functions that keep the intrinsic rate of nucleotide substitutions very low, but many viral polymerases have no editing functions. Editing refers to the process of excising a misincorporated nucleotide and replacing it with the correct nucleotide during replication. Because the viral polymerases lack editing functions, viruses that rely on viral polymerases accumulate mutations at a much higher rate per generation than cells do.

Viral RdRps are particularly mutagenic; they misincorporate the wrong base approximately 1 in 10,000 times, with a range of 1 in 1,000 to 1 in 100,000, depending on the RdRp. These values are between 10 and 10,000 times higher than the misincorporation rate of cellular DNA replication.

Recombination occurs when two viruses co-infect the same cell and an offspring virus contains a combination of genes that originate with both parents instead of just one parent.

These are larger-scale genetic changes than those typically classified as mutations. They may enable viruses to jump from species or result in a pandemic because an adaptive immune response against one of the parent viruses is not effective against the recombinant offspring.

These genetic variations are problematic for vaccine development. Moreover, every year manufacturers produce about 500 million doses of vaccine, so that the sheer volume of vaccine required means that vaccine manufacturers must begin many months before flu season. Vaccine manufacturers make an educated guess about which forms of influenza will be circulating during the following flu season. Sometimes their guess is on target, but other times there is a mismatch between the vaccine and the most prevalent form of influenza in any given year. This situation can even cause the alarming effect of discouraging the public from getting vaccinated at all. It is also possible for a virus to jump from animals to humans. When this occurs, the zoonotic strain is at first not adapted to humans. Although at first it may seem like such a virus would be unable to replicate in humans, sometimes the opposite is true and instead the virus replicates so ferociously that the death rate is much higher than it is for human-adapted influenza strains.

Accordingly, techniques and methods for predicting virus mutations at the current and future time frame would be useful and highly sought after. A method that is capable to predict possible new genetic variants of viruses and which operates quickly and efficiently while minimizing the time necessary for discovery of new variants is desirable.

PCT/CN 2019/091652 A (THE CHINESE UNIVERSITY OF HONG KONG) 18.6.2019 r. relates generally to genetic epidemiology of viral infectious diseases (e.g., influenza) and in particular to measurement and prediction of virus genetic (or amino acid) mutation patterns for viruses that cause infectious diseases. Certain embodiments of the invention relate to techniques for measurement and prediction of virus mutation patterns based on viral sequences (e.g., amino acid sequences) and population epidemic level. The predictions are based on identifying an "effective mutation," i.e., a mutation (variation in an amino acid sequence or nucleic acid sequence) that contributes to the virus's evolutionary advantage over human immunity, as opposed to a "trivial mutation" that has no (or negligible) effect on the virus's ability to survive and reproduce.

### Summary of invention

The invention is defined by the appended independent claims. Additional features and advantages of the concepts disclosed herein are set forth in the description which follows, and in part will be obvious from the description, or may be learned by practice of the described technologies. The features and advantages of the concepts may be realized and obtained by means of methods particularly pointed out in the appended claims. These and other features of the described technology will become more fully apparent from the following description and appended claims, or may be learned by the practice of the disclosed concepts as set forth herein.

Genetic variation of viruses is a serious challenge for developing long term protective vaccines and treatments. A universal vaccine would protect against all strains of a virus by provoking broadly neutralizing antibodies. Hence, the need to be able to predict variations.

It is an objective, amongst others, of the present disclosure to alleviate the above drawback and to provide a computer-implemented method for predicting variations with artificial intelligence algorithms.

Artificial Intelligence networks are able to discover rich, hierarchical models that represent probability distributions over the kinds of data encountered in artificial intelligence applications. The invention involves adversarial nets a discriminator and a generator which are trained in a minimax two-player game mode. While the generator learns to generate real looking samples the discriminator learns to distinguish fake from real samples. When reaching equilibrium the generator is able to generate realistic unique sample that are representative of the latent features of the samples encountered in the training data.

The sampler is translating a random sample from the distribution of viral nucleic or amino acids considered as the current known gene or protein pool from a concrete or multiple viruses into the latent space by performing convolutions.

To add additional randomness, the method further involves a sample from the Gaussian probability distribution which is concatenated to the output produced by the sampler. The concatenation layer has trainable weights and is part of the backpropagation.

The output of the concatenation is fed into the generator that consists of stacked transposed convolution layer that up samples the input. The produced output by the generator is supposed to trick the discriminator by being as similar to a real viral nucleic or amino acid of a particular virus variant.

An additional step is added to the method disclosed under the form of a validator. The validator simulates the proofreading ability of some viruses and cells. The validator consists of a concatenator, encoder and decoder. The concatenator merges the generated fake nucleic or amino acid sequence with the real sample.

The concatenated input is fed into a convolutional encoder to down sample it and translate it into the latent space. The produced latent space features are fed into a convolutional decoder that up sample it to produce the final output.

The output of the decoder is the output of the validator and produces the new variant of the nucleic or amino acid sequence. The new variant is consumed by the convolutional discriminator that has to classify it as fake or a real variant of the virus.

When the generator and discriminator have achieved equilibrium by applying the minimax two-player game mode, the generator starts to produce realistic variants of the nucleic or amino acid sequence.

The following detailed description, together with the accompanying drawings, provides a better understanding of the nature and advantages of the claimed invention.

The invention allows for the prediction of possible new genetic variants of viruses and operates quickly and efficiently while minimizing the time necessary for discovery of new variants is desirable. The results of the prediction could be used in the design, optimization and assessment of the efficiency of vaccines.

### Brief description of drawings

In order to best describe the manner in which the above-described method is implemented, as well as define other advantages and features of the disclosure, a more particular description is provided below and is illustrated in the appended drawings. Understanding that these drawings depict only exemplary embodiments of the invention and are not therefore to be considered to be limiting in scope.

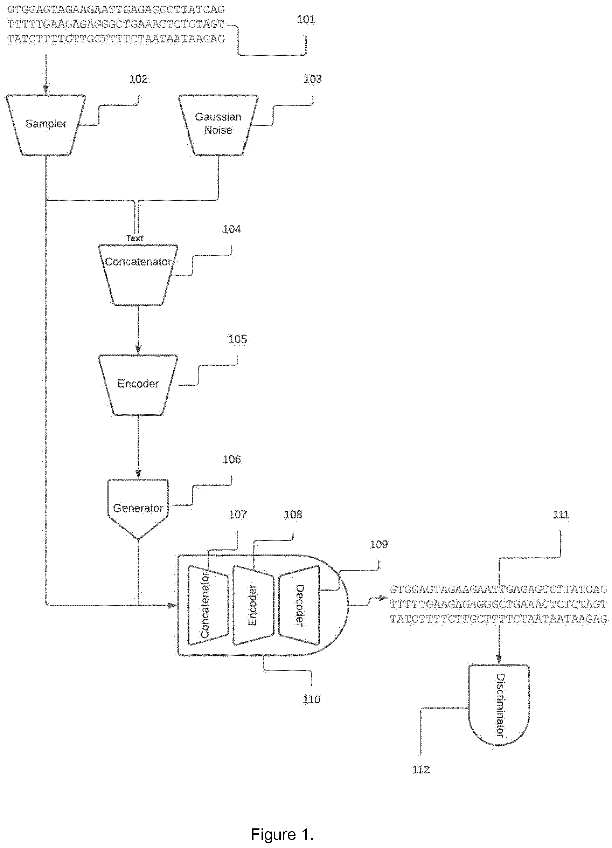

Fig. 1 illustrates an example method for predicting virus variants based on data from a nucleic or amino acid sequence, the model architecture and output.

### Description of embodiments

The disclosure relates to a computer-implemented method for generating variants of nucleic or amino acids through an adversarial generative neural network comprising of one or more deep learning blocks. Figure 1 illustrates such a network.

The generative adversarial network comprises of a convolutional sampler 102 that samples nucleic or amino acids 101. Noise is sampled 103 from a Gaussian distribution. The Concatenator 104 concatenates the outputs produced by 102 and 103 and feeds the result into the Encoder 105. The task of the encoder is to translate the input into the latent space by reducing the dimensions through convolution computations. The Generator 106 ingests the output from 105 as input and produces the prediction by up sampling the latent features through transposed convolutional computations.

The produced output is fed into the Validator 110 that consist of a Concatenator 107 that concatenates the produced variant, Encoder 108 that translates the merged data into the latent space and Decoder 109 that produces a final prediction 111 of a variant of nucleic or amino acids.

The Discriminator 112 has to classify 111 as being real or fake.

Data analysis and computational operations described herein can be executed in computer systems of such as server, web server, desktop computer, laptop computer, tablet, mobile device (e.g., smart phone), or the like. High performance computing, computer clusters and/ or cloud-based computing can used. Such systems may include one or more machines or servers composed of one or more processors to execute program code (e.g., general-purpose microprocessors used as a central processing unit (CPU) and/or one or more special-purpose processors such as graphics processors (GPUs) that my provide enhanced parallel-processing capability; computer programs incorporating various features of the invention may be encoded and stored on various computer readable storage media.

The herein described invention with reference to specific embodiments is applicable under different variations and modifications. All blocks and processes described above are illustrative and may be modified. Operations described as blocks can be combined or used independently. Order of operations, block arrangements and data flow may be modified to the extent logic permits. Processes described above can be altered or omitted and additional processes or blocks can be not specifically described may be added. Particular definitions and data formats can be modified as desired.

Further, while embodiments described above refer specially to virus gene modifications those skilled in the art will appreciate that the same analytical approach can be applied to other living or not living forms that have the ability to mutate, and the invention is not limited to a particular type of living or not living type.

### Industrial applicability

The method described herein can be applied to any sequence of epidemic data of one or multiple virus mutations with optional application of mathematical combination of regional and global data. In respect to the mathematical combinations applied on the data the generated candidate virus can be specific to the parameters used for the combination.

The method described herein can be applied on any or all gene or protein segments of one or multiple viruses that cause infectious diseases.

The method described herein can be applied on any virus subtypes or on or in combination with new emerging pathogens that may cause or a causing epidemics or pandemics.

The method described herein can be employed in a computer-implemented method for predicting virus mutations. Inputs to the process can be data collected during a period of time including sequence data of the genes or proteins of one or multiple viruses that cause infectious diseases.

The predictive methods described can be used for instance for predicting future pandemic. The virus can be predicted by combining human with other species sequence data. Future effective mutations can be predicted. Future virus protein or genome sequence can be predicted. Vaccine match scoring can be calculated from the difference between the predicted virus and the mutations included in the vaccine.

Predictions of a virus 111 made by the Generator 109 can be interpreted by medical professionals for multiple uses. Examples include: selecting virus strains to include in a vaccine; analysing possible gene mutations for the creation of universal virus vaccine; assessing the effectiveness of currently available vaccine or treatment; identifying parts of gene or protein sequences that are mutagenic.

Although the invention is described with respect to the above mentioned embodiments, those skilled of in the art will appreciate that the invention is intended to cover all the modifications and equivalents within the scope of the described claims.

**Reference signs list**

**Reference to deposited biological material**

**Sequence listing free text**

**Citation list**

**Unrecognised text**

## Claims

1. A computing system for predicting nucleic or amino acids variations of viruses wherein the system comprises:
- a sampler module (102) configured to sample from a distribution of genetic variants, proteins of a particular or multiple viruses; and
- a concatenator module (104) configured to produce an output by concatenating the sampled genetic variant with noise sampled from Gaussian probability distribution (103); and
- a encoder module (105) configured to translate the concatenated input into the latent space by computing convolutions on it; and
- a generator module (106) configured to up-sample the latent features to a realistically looking variation of nucleic or amino acids; and
- a validator module (110) configured to mimic the proof reading capability of viruses and cells that comprises of:
- a concatenator module (107) configured to merge the generated material with the real one; and
- a encoder module (108) configured to down sample the merged output into the latent space; and
- a decoder module (109) configured to up sample the latent features to a realistic nucleic or amino acid variation (111)
;and
- a discriminator module (112) configured to classify the generated variation (111) as fake or real.

2. The method according to claim 1, wherein the generator (106) seeks to achieve equilibrium with the discriminator (112) by applying a minimax two-player game mode.

3. A computer-implemented method for predicting viral genetic variants utilising the system according to any one of clams 1 to 2, wherein data from previously known genetic variants, proteins of a virus or multiple viruses is used to train the system to produce new variants of the gene or the protein of the virus.

4. A computer program product comprising instructions which, when the program is executed by a computer, cause the computer to perform the method any one of claims 1 to 3.

5. A computer readable storage medium comprising the computer program product according to any one of claims 1 to 4.
